# EUROPÄISCHE PATENTSCHRIFT

(11) **EP 1 951 870 B1**
(45) Veröffentlichungstag und Bekanntmachung des Hinweises auf die Patenterteilung: **24.04.2013**
(21) Anmeldenummer: 06805529.2
(22) Anmeldetag: 22.11.2006
(51) Int. Cl.: C12N 15/113, C12N 15/10

(54) **DNA-KONSTRUKTE ZUR SPEZIFISCHEN HEMMUNG DER GENEXPRESSION DURCH RNA-INTERFERENZ**
DNA CONSTRUCTS FOR SPECIFIC INHIBITION OF GENE EXPRESSION BY RNA INTERFERENCE
PRODUITS DE RECOMBINAISON DE L'ADN POUR L'INHIBITION SPECIFIQUE DE L'EXPRESSION DE GENES PAR INTERFERENCE ARN

(30) Priorität: 25.11.2005 EP 05025727
(43) Veröffentlichungstag der Anmeldung: 06.08.2008
(73) Patentinhaber: Mologen AG, 14195 Berlin (DE)
(72) Erfinder: SCHROFF, Matthias, 13465 Berlin (DE); OSWLAD, Detlef, 10999 Berlin (DE)
(74) Vertreter: Tegethoff, Sebastian
(86) Internationale Anmeldenummer: PCT/DE2006/002083
(87) Internationale Veröffentlichungsnummer: WO 2007/059760

(56) Entgegenhaltungen:
- WO-A-98/21322
- WO-A-2004/016786
- WO-A-2004/044135
- WO-A-2005/116223
- US-A1- 2004 053 876

## Beschreibung

Die Erfindung betrifft Verfahren zur Herstellung von Vektoren, die geeignet sind, nach ihrer Transfektion in eukaryote Zellen gezielt die Bildung von einem oder mehreren Proteinen durch RNA-Interferenz zu inhibieren sowie derartige Vektoren.

Eine kürzlich nachgewiesene Möglichkeit der Hemmung der Genexpression beruht auf der Erzeugung von doppelsträngigen RNA-Moleküle. Mit dieser Doppelstrang-RNA (dsRNA) lassen sich hochwirksam und schneller als mit jedem anderen Verfahren einzelne Gene gezielt ausschalten, ohne die Proteinbildung benachbarter Gene zu stören. Das zugrunde liegende Prinzip wird als RNA-Interferenz, kurz RNAi, bezeichnet. Die dieses Phänomen verursachende dsRNA-Sequenz als siRNA (small interference RNA).

Die siRNA verhindert nicht das Ablesen des Gens, sondern schaltet einen zelleigenen Mechanismus an, der die vom Gen abgelesenen mRNA's und so die Bildung des entsprechenden Proteins unterbindet (post-transcriptional gene silencing).

Ausgelöst wird dieser gezielte mRNA-Abbau durch kurze siRNA-Moleküle mit 19-23 RNA-Basen Länge, die homolog zu der Target-mRNA sind, deren Umsetzung in ein Protein verhindert werden soll. Die siRNA-Moleküle setzen sich mit speziellen Endoribonukleasen zu einem zelleigenen RNA-Proteinkomplex mit der Bezeichnung "RISC" (RNA-induced silencing complexes) zusammen. Bei dem Aufbau dieser Komplexe dissoziieren die beiden RNA-Stränge voneinander, wodurch sogenannte aktivierte RISC entstehen, die jeweils einen Einzelstrang des siRNA-Moleküls enthalten. Aktivierte RISC, welche den antisense-Strang enthalten, der komplementär zu der Target-mRNA ist, binden an diese und die Endoribonuklease des RNA-Proteinkomplexes sorgt nun für den sequenzspezifischen mRNA-Abbau.

Die siRNA kann in der Zelle experimentell erzeugt werden oder durch Einschleusen von außen eingebracht werden. Zum Einen gelingt das über synthetisch hergestellte siRNA-Moleküle, die sowohl in-vitro und in-vivo verabreicht werden können.

Diese Methode hat jedoch technische Grenzen. Neben der generellen Instabilität der synthetischen siRNA im Medium als auch in der Zelle, ist die Inhibierung mittels synthetischer siRNA prinzipiell nur vorübergehend möglich und viele Zellen (z.B. neuronale Zellen) lassen sich nur sehr ineffizient transfizieren. Studien, die auf der Transfektion synthetischer siRNA beruhen, sind daher in der Regel sowohl zeitlich auf 1 - 5 Tage als auch Zelltyp-spezifisch eingeschränkt, Im Weiteren sind die hohen Produktionskosten und die lange Produktionsdauer nachteilig.

Die WO 2004/044135 offenbart Oligomerverbindungen für die Modulation von Genexpression mittels RNA-Interferenz. Die Oligomerverbindungen umfassen dabei zwei Oligomere, die mindestens teilweise miteinander hybridisieren und von denen eins wiederum mit Target-Nukleinsäuren hybridisieren kann, um die Genexpression zu unterdrücken. Mindestens eines der Oligomere hat eine nicht-lineare Sekundärstruktur oder ist Teil einer multiplen Oligomeranordnung.

siRNA kann jedoch auch durch Vektoren in der Zelle erzeugt werden. Dabei handelt es sich um virale oder plasmidbasierte Vektoren, die erst in der Zelle durch Expression zur Bildung der siRNA-Sequenzen führen. Die Vorteile gegenüber der Transfektion mit synthetischer siRNA liegen in der stabileren und ggf. regulierteren Transkription der entsprechenden siRNA-Sequenz.

Jedoch zeigen die plasmidbasierten Vektoren neben einer niedrigen Transfektionseffizienz ebenfalls einen aufwendigen Produktionsprozess. So ist es notwendig stabile Klone zu selektieren. In diesem oftmals langwierigen Prozess, der Wochen aber auch Monate dauern kann, kommt es häufig zum Auftreten zahlreicher potentieller Schwierigkeiten, die Klonierungsexperimenten innewohnen. Zur Überprüfung des Produkts sind Sequenzierungen notwendig, die ebenfalls arbeits- und kostenintensiv sind.

Des Weiteren enthalten die plasmidbasierten Vektoren Antibiotika-Resistenzgene, die zu ihrer Selektion notwendig sind. Aus diesem Grund sind derartige Vektoren nicht zur Anwendung in lebenden Organismen geeignet. Die mögliche Rekombination mit ubiquitär im Organismus vorkommenden Bakterien, birgt das Risiko eines zunehmenden Auftretens antibiotikaresistenter Bakterien. Die Verbreitung von Antibiotikaresistenzen ist ein schwerwiegendes Problem und nicht vertretbar.

Da virale Vektoren zur effizienten und gezielten Transfektion fähig sind, bieten sie einen Vorteil gegenüber synthetischen siRNA-Molekülen als auch plasmidbasierten Vektoren.

Für die therapeutische Anwendung sind derartige virale Vektoren jedoch nur mit Vorbehalt einsetzbar. Auch hier stellt die Rekombination viraler Sequenzen mit natürlich vorkommenden Viren ein inhärentes Sicherheitsrisiko dar, da die Erzeugung neuer, pathogener Hybridviren befürchtet werden muss. Zudem ist auch ihre Herstellung aufwendig und kostenintensiv.

Da schon allein die Inhibition der Expression von nur einem Genprodukt unter der Verwendung von Expressionssystemen mit einer Vielzahl von Komplikationen verbunden ist, versteht es sich nahezu von selbst, dass die simultane Abschaltung der Expression mehrerer Genprodukte in einer Zelle oder einem Gewebe deutlich komplexer und somit schwieriger zu erzielen ist.

Eines der Hauptprobleme bei der multiplen Hemmung der Genexpression durch RNA-Interferenz, für den Fall der Verwendung mehrerer unabhängiger Konstrukte, stellt die geringe Wahrscheinlichkeit dar, dass eine Zelle allein von nur einem Konstrukt transfiziert wird. Diese geringe Wahrscheinlichkeit potenziert sich negativ mit der Anzahl an verwendeten Konstrukten. Oftmals ist jedoch für bestimmte gentherapeutische Ansätze eine kontrollierte, simultane Hemmung der Genexpression erforderlich.

Es existiert eine Vielzahl an Methoden, welche die Kotranskription mehrerer RNAs in einer Zelle ermöglichen. Die einfachste Methode ist die Kotransfektion zweier unabhängiger Expressionskonstrukte (im Folgenden auch: Vektoren). Eine andere Methode besteht in der Transfektion mit einem Vektor, der zwei unabhängige Expressionskassetten trägt. Derartige Konstrukte lassen sich auch für die Erzeugung von siRNAs verwenden.

Bei beiden Möglichkeiten besteht jedoch die Gefahr, dass die Transkripte sich signifikant bezüglich ihrer Anzahl, Prozessierung, Halbwertszeit und translationalen Effektivität und somit in der Menge des exprimierten Proteins unterscheiden, so dass ihr Einsatz durch Ineffektivität und schlechte Reproduzierbarkeit gekennzeichnet ist. Daher würde die Verwendung derartiger Expressionssysteme auch zu einer unterschiedlich starken Transkription von siRNA führen, wodurch letztlich eine ungleiche Hemmung der jeweiligen Gene erreicht würde.

Die Konstruktion von di- und polycistronischen RNAs durch die Verwendung von IRES (Internal Ribosome Entry Site) Elementen stellt eine weitere Möglichkeit zur Koexpression mehrerer Gene dar. Diese ermöglichen die Initiation der Translation durch Ribosomen unabhängig von der mRNA-cap-Struktur durch Sequenzelemente. Erstmalig wurden IRES-Elemente in der mRNA des Picornavirus entdeckt (Pelletier und Sonenberg, 1988, Nature 334: 320-325, Jang et al., 1988, Journal Virology 62: 2636-2643).

Für die Konstruktion bicistronischer Vektoren werden am häufigsten die IRES-Elemente des Poliovirus und des EMCV (Encephalomyocarditis Virus) verwendet (Dirks et al., 1993, Gene 128: 247-249). Nachteilhafterweise variiert deren Effektivität stark in Abhängigkeit von der verwendeten Zelllinie (Borman et al., 1997, Nucleic Acids Res. 25: 925-932).

Die meisten der verfügbaren bicistronischen Expressionskassetten bestehen aus einem selektierbaren Marker oder einem Reportergen, welche auf der 3'-Seite des IRES Elementes und einer MCS (Multiple Cloning Site) für die Einfügung des gewünschten Gens angeordnet sind (Dirks et al., 1993, Gene 128: 247-249). Ebenso sind tri- und polycistronische Expressionssysteme unter Verwendung von IRES-Elementen bekannt (Zitvogel et al., 1994, Hum Gene Ther 5: 1493-1506, Fussenegger et al., 1998a, Nature Biotechnol. 16: 468-472, Mielke et al., 2000, Gene 254. 1-8).

Für die therapeutische Anwendung sind virale IRES-Elemente jedoch nur mit Vorbehalt einsetzbar. Die Rekombination viraler Sequenzen mit natürlich vorkommenden Viren stellt auch hier ein inhärentes Sicherheitsrisiko dar, da die Erzeugung neuer, pathogener Hybridviren befürchtet werden muss.

Multicistronische Vektoren können auch durch die Verknüpfung von Transkriptionseinheiten ohne IRES-Elemente konstruiert werden. Zum Einen können die gewünschten Gene über verschiedene MCS in einen Plasmid-Vektor kloniert werden, und zum Anderen können aus Plasmiden isolierte Expressionskassetten durch DNA-Linker zu linearen multicistronischen Vektoren verknüpft werden (Tsang et al., 1997, Bio Techniques 22: 68).

Untersuchungen der Expressionsraten linearer cis-verknüpfter Gene zeigten jedoch einen starken negativen Einfluss auf die Transkription derart angeordneter Expressionskassetten (Esperet et al., 2000, J Biol Chem 275: 25831-25839).

Plasmid-Vektoren für die Expression multipler Gene unterscheiden sich von den üblichen Plasmid-Vektoren durch die Anzahl der klonierten Transkriptionseinheiten. Dabei können Promotoren bzw. poly(A)-Sequenzen gleichen oder unterschiedlichen Ursprungs für die Transkriptionseinheiten verwendet werden.

Der Nachteil, der sich aus der Verwendung unterschiedlicher Promotoren ergibt, besteht darin, dass die Promotoren sich in ihrer Stärke unterscheiden und so die Expressionsraten der einzelnen Gene variieren können, wohingegen der Einsatz gleicher Promotoren zur Ausbildung von Sekundärstrukturen der DNA führen kann, welche einen Funktionsveriust der Promotoren zur Folge haben können,

Allen genannten Vektoren zur multiplen Genexpression ist gemeinsam, dass sie entweder auf Plasmiden oder aber viralen Vektoren basieren. Neben allen beschriebenen Unzulänglichkeiten der gegenwärtigen mehrfachkodierenden Vektoren weisen sie zusätzlich die Nachteile dieser Art von Vektoren auf. Die Nachteile viraler Vektoren, zu denen Instabilität des attenuierten Impfstammes zählen, und die Unzulänglichkeiten von Plasmid-DNA-Vektoren, wie die mit ihrer Verwendung einhergehende Verbreitung von Antibiotikaresistenzgenen (ausführlich beschrieben in EP 0 941 318 B1), sind hinlänglich bekannt.

Die US 2004/0053876 offenbart haarnadelförmige siRNA-Strukturen sowie DNA kodierend für haamadelförmige siRNA-Strukturen zur Unterdrückung der Genexpression sowie deren Herstellung. Es ist auch möglich, dass mehr als eine Haarnadei-siRNA in einem RNA-Konstrukt vorhanden ist. Als DNA-Konstrukte werden Plasmide und virale Vektoren aufgeführt. Daher weisen sie die bekannten Nachteile dieser DNA-Konstrukte auf (s.o.).

Die EP 0 941 318 B1 (aus WO 98/21322) betrifft ein expressionsfähiges Nukleinsäurekonstrukt, welches nur die zur Expression eines Gens zur RNA- oder Proteinsynthese notwendige Sequenzinformation enthält. Solche Expressionskonstrukte können in der Gentherapie und genetischen Vakzination eingesetzt werden und vermeiden viele mit den gängigen Konstrukten verbundenen Risiken. Jedoch kodieren diese Konstrukte jeweils nur für ein Gen oder eine RNA.

Wünschenswert wären daher Expressionssysteme für siRNA, die auch zur simultanen Hemmung der Genexpression mehrerer Gene in der Lage sind. Das wohl größte Problem bei der Lösung dieser Fragestellung stellt eine Verbindung (Linker) der Sequenzen zur Erzeugung der siRNAs dar, welche eine jeweils ausreichende Transkription zur Hemmung der Genexpression dieser Sequenzen gewährleistet. Eine Methode zur Verknüpfung von DNA-Fragmenten durch DNA-Verbindungselemente ist aus der Literatur bekannt. Seeman beschreibt DNA-Sequenzen zur Untersuchung von natürlich vorkommenden Verzweigungen, sog. Holliday-Junctions, die als topologische Sonderformen von B-DNA unter torsionalem Stress postuliert werden (Seeman: 1982, J Theor Biol 99: 237-247; Annu. Rev. Biophys. Biomol. Struct. 1998. 27:225-248). Die Anwendung dieser Strukturen blieb bisher auf kurze Oligonukleotid-Stücke zur Untersuchung von DNA-Strukturformen beschränkt.

Die WO 2004/016786 betrifft ein DNA-Expressionskonstrukt zur multiplen Genexpression, ferner ein Verfahren zu seiner Herstellung und ein Verbindungsmolekül (Linker) zur Verknüpfung von DNA-Expressionskassetten zur Herstellung eines DNA-Expressionskonstruktes zur multiplen Genexpression, sowie letztlich Verwendungsmöglichkeiten in der genetischen Vakzinierung. Jedoch werden hier verschiedene, aber vollständige Expressionskassetten verknüpft, d.h. jede Expressionskassette hat ihren eigenen Promotor und ihre eigene kodierende Sequenz. Diese werden über Holliday-Strukturen miteinander verknüpft.

Aus der DE 100 48 417 A1 ist eine polyfunktionale Verbindung bekannt, welche die Konjugation von zwei reaktiven funktionellen Gruppen mittels eines Linkers beschreibt. Als biologische Substanzen, welche derart gekoppelt werden können, werden auch Nukleinsäuren erwähnt, die Beispiele zeigen jedoch nur die Anwendungen der Erfindung zur Konjugation von Peptiden. Einen anderen Ansatz mehrere Gene zur effizienten Expression in Zellen einzuschleusen beschreibt die WO 01/87348 A2. Nukleinsäuren sollen durch kovalente Bindungen zu supramolekularen Strukturen, sog. Dendrimeren, verknüpft werden.

Eine weitere Möglichkeit Vektoren für siRNA herzustellen, zeigt http://www.ambion,com. Dieser Prozess vermeidet die oben genannten Nachteile. Jedoch ist auch dieser Produktionsprozess zeitaufwendig und durch eine Reihe an notwendigen Vervielfältigungsschritten der betreffenden Sequenzen mittels PCR (polymerase chain reaction) sehr fehlerbehaftet. Die Möglichkeit der Erzeugung sowohl unerwünschter als auch unbemerkter Mutationen, die durch den PCR-Prozess noch potenziert werden, ist sehr groß. So sind auch hier Kontrollsequenzierungen nötig, die den Herstellungsprozess verlängern und zu erhöhten Kosten beitragen.

Ausgehend von diesem Stand der Technik ist es Aufgabe der Erfindung ein geeignetes Verfahren zur in-vitro oder in-vivo Synthese einer oder mehrerer definierter siRNA-Sequenzen, die dadurch hergestellten Expressionskonstrukte sowie einen Kit zur Durchführung der Synthese zur Verfügung zu stellen.

Gelöst wird diese Aufgabe durch die Merkmale der selbstständigen Ansprüche.

Erfindungsgemäß ist danach ein Expressionskonstrukt vorgesehen, dass aus einer ersten DNA-Abschlusshaarnadelstruktur besteht, bei der ein Einzelstrang aus Desoxyribonukleotiden derart zu einem doppelsträngigen Bereich rückgefaltet ist, dass dieser ein Ende mit kohäsivem Überhang aufweist und am entgegengesetzten Ende die den Doppelstrang bildenden Einzelstränge durch einen einzelsträngigen Loop miteinander verbunden sind, einer zweiten Abschlusshaarnadelstruktur, die analog zur ersten Abschlusshaarnadelstruktur aufgebaut ist und einer T-förmigen DNA-Struktur mit drei doppelsträngigen DNA-Armen, welche über Holliday-Junctions verbunden sind, wobei wenigstens ein Arm die zu transkribierenden Sequenzen enthält und die Einzelstränge dieses DNA-Doppelstrangarmes an dem Ende, welches der Verbindung mit den anderen Armen entgegengesetzt ist, durch einen einzelsträngigen DNA-Loop miteinander verbunden sind, und die beiden anderen Arme kohäsive Enden aufweisen, wobei ein Arm eine geeignete Terminationssequenz aufweist und der andere Arm eine geeignete Promotorsequenz.

Die genannten Komponenten stellen die essentiellen Bestandteile eines erfindungsgemäßen Konstruktes dar. Von der Erfindung sind auch Expressionskonstrukte umfasst, welche diese Komponenten aufweisen, wobei diese jedoch aus anderen Kombinationen von DNA-Strukturen hervorgehen.

Erfindungsgemäß ist weiterhin ein Expressionskonstrukt zur gezielten Hemmung der Genexpression mittels RNA-Interferenz vorgesehen, welches eine erste DNA-Abschlusshaarnadelstruktur, bei der ein Einzelstrang aus Desoxyribonukleotiden derart zu einen doppelsträngigen Bereich rückgefaltet ist, dass dieser ein Ende mit kohäsivem Überhang aufweist und am entgegengesetzten Ende die den Doppelstrang bildenden Einzelstränge durch einen einzelsträngigen Loop miteinander verbunden sind aufweist, ferner eine zweite Abschlusshaarnadelstruktur, die analog zur ersten Abschlusshaarnadelstruktur aufgebaut ist, und deren doppelsträngiger und einzelsträngiger Bereich aus der zu transkribierenden Sequenz besteht sowie einer geeigneten Promotorsequenz und einer geeigneten revers-komplementären Terminationssequenz, die sich zwischen den beiden oben beschriebenen Abschlusshaarnadelstrukturen in einem DNA-Doppelstrang befinden.

Ein derartiges Expressionskonstrukt weist nur eine doppelsträngige Kopie der zu erzeugenden siRNA auf. Die Erzeugung einer siRNA- bei Vorliegen nur einer Kopie ist mit diesem Konstrukt möglich, da die zu transkribierenden Sequenzen von der RNA-Polymerase über den einzelsträngigen Loop hinweg abgelesen und transkribiert werden. Dies wäre mit einem doppelsträngigen Plasmid nicht möglich.

Für beide erfindungsgemäßen Expressionskonstrukte ist vorgesehen, dass mehrere T-förmige oder lineare DNA-Strukturen jeweils an einem Ende durch einen Linker mittels Holliday-Junctions verbunden sind. In diesem Fall ist vorgesehen, dass die zu transkribierenden Sequenzen für identische oder unterschiedliche Gene kodieren.

Unter Linker sollen Oligonukleotide zur Verbindung von DNA-Expressionskassetten verstanden werden. Ein erfindungsgemäßer Linker besteht aus zwei oder mehr Strängen, wie an folgendem Beispiel für einen Linker, gebildet aus drei Oligonukleotiden, die mit drei 3 Expressionskassetten auf miteinander verbundenen Strängen A, B und C verbunden sind, illustriert werden soll: Das 5'-Ende von A hat zu dem 3'-Ende von B eine komplementäre Sequenz und bildet einen Doppelstrang, während das 3'-Ende von A mit dem 5'-Ende von C paart, und schließlich das 5'-Ende von B mit dem 3'-Ende von C paart. Diese Art von Verbindung von DNA-Doppelsträngen wird auch als Holliday-Junction bezeichnet (F.W. Stahl: Genetics. 1994 Oct;138(2):241-6), welche den Vorteil bietet, dass ausschließlich natürlich vorkommende Bausteine zum Verbinden der Expressionskassetten verwendet werden, was sich bei einer Verwendung der Erfindung zu medizinischen Zwecken aus toxikologischen Gründen als besonders vorteilhaft erweisen kann.

Die für die DNA-Verzweigungen verwendeten Oligodesoxynukleotidsequenzen werden so gewählt, dass bei der Hybridisierung an den 5'-Enden der Oligonukleotide (beispielsweise) je ein vier Basen langes, überhängendes, kohäsives Ende erzeugt wird. Die Sequenzen der kohäsiven Enden können sich für jeden Arm einer Verzweigung unterscheiden, müssen es aber nicht. Die T-förmigen oder linearen DNA-Strukturen weisen ein zu dem kohäsiven Ende komplementären Überhang auf. Hierdurch wird die gezielte Ligation der Expressionskassetten an die einzelnen "Arme" der Verzweigungen möglich.

Derartig ausgestaltete erfindungsgemäße Expressionskonstrukte sind zur gezielten, multiplen Hemmung der Genexpression verschiedener Gene geeignet. Aufgrund des identischen Aufbaus der T-förmigen Strukturen, welche jeweils die zur Erzeugung der siRNAs notwendigen Sequenzen enthalten, werden die verschiedenen siRNAs gleich stark erzeugt. Die Anzahl der Gene, deren Proteinexpression gehemmt wird, ist abhängig von der Anzahl der über den Linker miteinander verbundene T-förmigen Strukturen.

Vorteilhafterweise sind in einer alternativen Ausführungsform des erfindungsgemä-βen Expressionskonstruktes die kohäsiven Enden einzelner Komponenten zur gezielten Verbindung mit anderen Komponenten spezifisch und unterschiedlich gewählt. Dadurch wird der gezielte Aufbau des gewünschten Expressionskonstruktes ermöglicht.

Ein DNA-Expressionskonstrukt, das die Sequenzen zur Erzeugung einer oder mehrer siRNAs enthält, kann Zellen gezielt transfizieren.

Unter Transfektion soll das Einbringen von Nukleinsäuresequenzen mittels biologischer, chemischer oder physikalischer Methoden in die Zelle verstanden werden, in dessen Folge es zu anhaltender oder vorübergehender Expression von durch diese Sequenzen kodierten katalytisch wirksamen RNA-Transkripten in der transfizierten Zelle kommt.

Die erfindungsgemäßen Expressionskonstrukte weisen in einer Weiterbildung kovalent gebunden ein oder mehrere Peptide, Proteine, Kohlenhydrate, Antikörper oder Steroide auf.

Ein weiterer Gegenstand der vorliegenden Erfindung sind Herstellungsverfahren für Expressionskonstrukte, die nach ihrer Transfektion in eukaryote Zellen die Bildung definierter Proteine gezielt durch RNA-Interferenz inhibieren.

Für lineare Expressionskonstrukte ist ein Verfahren vorgesehen, beginnend in Verfahrensschritt a) mit der Mischung eines DNA-Doppelstranges, welcher eine 19 - 23 Basen lange, singuläre Kopie einer Gensequenz oder ihre revers-komplementäre Sequenz sowie ein Terminationssignal für RNA-Polymerasen enthält und durch einen 8 - 12 Basen langen Sequenzloop an einem Ende abgeschlossen ist, welcher so gewählt ist, dass gegenüberliegende Basen in keinem Fall komplementär zueinander sind und die flankierenden DNA-Einzelstränge zu einem DNA-Doppelstrang miteinander verbunden sind, der am anderen Ende einen kurzen überstehenden DNA-Einzelstrang aufweist, mit einem haarnadelförmigen Oligodesoxynukleotid, welches am Ende kurze überstehende Enden einzelsträngiger DNA aufweist in Verfahrensschritt b) und in Verfahrensschritt c) einem Promotor mit kurzen überstehenden Enden einzelsträngiger DNA, wobei das einzelsträngige 5'-Ende des Promotors mit dem haarnadelförmigen Oligodesoxynukleotid oder dem 10 bis 1000 basenlangen Doppelstrang paaren kann und das einzelsträngige 3'-Ende des Promotors komplementär zu dem einzelsträngigen 5'-Ende des unter a) beschriebenen DNA-Doppelstranges ist und anschließender Ligation der DNA-Fragmente in Verfahrensschritt d) sowie abschließender Aufreinigung der hergestellten Vektoren im letzten Verfahrensschritt e).

Optional ist das erfindungsgemäße Verfahren zur Herstellung linearer Expressionskonstrukte derart ausgestaltet, dass vor Zugabe des haarnadelförmigen Olidodesoxynukleotids in Verfahrensschritt c) ein 10 bis 1000 Basen (= n) langen DNA-Doppelstrang einer nichtkodierenden Sequenz mit kurzen überstehenden Enden einzelsträngiger DNA zugegeben wird, wobei das einzelsträngige 3'-Ende mit dem einzelsträngigen 5'-Ende des Promoters paaren kann und das einzelsträngige 5'-Ende komplementär zu dem einzelsträngigen 3'-Ende des haarnadelförmigen Oligodesoxynukleotid ist.

Zur Herstellung von T-förmigen Expressionskonstrukten, die nach ihrer Transfektion in eukaryote Zellen die Bildung definierter Proteine gezielt durch RNA-Interferenz inhibieren, ist ein Verfahren vorgesehen, beginnend in Verfahrensschritt a) mit der Mischung eines T-förmigen DNA-Doppelstranges, welcher eine 19 - 23 Basen lange, singuläre Kopie einer Gensequenz in 5' - 3'-Richtung sowie ein Terminationssignal für RNA-Polymerasen enthält und durch einen 8 -12 Basen langen Sequenzloop an einem Ende abgeschlossen ist, welcher so gewählt ist, dass gegenüberliegende Basen in keinem Fall komplementär zueinander sind und die flankierenden Doppelstrangbereiche so durch zwei DNA-Einzelstränge miteinander verbunden sind und am anderen Ende zwei kurze überstehende DNA-Einzelstränge aufweist, und einem Oligodesoxynukleotid dessen Sequenz komplementär zu den beiden kurzen überstehenden DNA Einzelsträngen ist und zwei kurze überstehende Enden einzelsträngiger DNA bildet, in Verfahrensschritt b) mit einem haarnadelförmigen Oligodesoxynukleotid, welches am Ende kurze überstehende Enden einzelsträngiger DNA aufweist und in Verfahrensschritt c) einem weiteren haarnadelförmigen Oligodesoxynukleotid, welches am Ende kurze überstehende Enden einzelsträngiger DNA aufweist, komplementär zu dem T-förmigen DNA-Doppelstrang aus a) und in Verfahrensschritt d) einem Promotor mit kurzen überstehenden Enden einzelsträngiger DNA, wobei das einzelsträngige 5'-Ende des Promotors mit dem haarnadelförmigen Oligodesoxynukleotid aus b) paaren kann und das einzelsträngige 3'-Ende des Promotors komplementär zu dem einzelsträngigen 5'-Ende des T-förmigen DNA-Doppelstranges ist, gefolgt von anschließender Ligation der DNA-Fragmente in Verfahrensschritt e), wobei das Konstrukt auch ohne den 10 bis 1000 Basen langen DNA-Doppelstrang hergestellt werden kann sowie abschließender Aufreinigung der hergestellten Vektoren im abschließenden Verfahrensschritt f).

Dieses Herstellungsverfahren weist erfindungsgemäß nach Verfahrenschritt c) die Zugabe eines 10 bis 1000 Basen (= n) langen DNA-Doppelstrang einer nichtkodierenden Sequenz mit kurzen überstehenden Enden einzelsträngiger DNA auf, wobei das einzelsträngige 3'-Ende mit dem einzelsträngigen 5'-Ende des Promoters paaren kann und das einzelsträngige 5'-Ende komplementär zu dem einzelsträngigen 3'-Ende des haarnadelförmigen Oligodesoxynukleotid aus Verfahrensschritt b) ist zugegeben wird und/oder die Zugabe eines 10 bis 1000 Basen (= n) langen DNA-Doppelstranges einer nichtkodierenden Sequenz mit kurzen überstehenden Enden einzelsträngiger DNA, wobei das einzelsträngige 5'-Ende mit dem einzelsträngigen 3'-Ende des T-förmigen DNA-Doppelstranges paaren kann und das einzelsträngige 3'-Ende komplementär zu dem einzelsträngigen 5'-Ende des haarnadelförmigen Oligodesoxynukleotid aus Verfahrensschritt c) ist.

In einer bevorzugten Ausführungsform ist ein Verfahren vorgesehen, bei dem der Promotor Teil eines bakteriell amplifizierbaren Plasmides ist, welches vor der Mischung der Komponenten mit einer Restriktionsendonuklease ausgeschnitten wird, welche eine den Promotor auf dem Plasmid flankierende Schnittstelle erkennt, die nicht auf dem herzustellenden Molekül vorhanden ist.

Grundsätzlich sind als Promotoren jegliche Promotoren vorgesehen, die in den Zielzellen, also den Zellen, in welchen die Genexpression gehemmt werden soll, operabel sind. Vorzugsweise sind diese Promotoren humanen Ursprungs, sofern humane Zellen oder Gewebe mit den erfindungsgemäßen Expressionskonstrukten transfiziert werden. Sie können jedoch beispielsweise ebenso viralen, bakteriellen oder parasitären Ursprungs sein oder aus anderen Spezies als der der Zielspezies stammen. Dabei sind als Promotor insbesondere, aber nicht ausschließlich, der menschliche U6-Promotor, der menschliche H1-Promotor, der murine U6-Promotor, der CMV-Promotor und der 7SK-Promotor vorgesehen.

Ferner ist es erfindungsgemäß vorgesehen, dass im Falle der Verwendung eines Promotors als Teil eines bakteriell amplifizierbaren Plasmides der Ligationsschritt in Anwesenheit der Restriktionsendonuklease erfolgt, mit welcher der Promotor aus dem Plasmid ausgeschnitten wurde.

In einer Ausführungsform wird vor dem abschließenden Aufreinigungsschritt ein Verdau der Reaktionsmischung mittels einer ausschließlich für 3'- oder 5'-DNA-Enden spezifischen Exonuklease durchgeführt.

Bei dem erfindungsgemäßen Verfahren kann der DNA-Doppelstrang, welcher zu Beginn der Mischung zugegeben wird, aus dem Annealing von einem partiell selbstkomplementären Oligodesoxynukleotid resultieren oder von zwei komplementären Oligodesoxynukleotiden. Das Annealing kann auch erst in der Reaktionsmischung erfolgen, so dass zu Beginn des erfindungsgemäßen Verfahrens lediglich einzelsträngige komplementäre Oligodesoxynukleotide zugegeben werden.

Die Sequenz der Oligodesoxynukleotide ist in einer bevorzugten Ausführungsform so gewählt, dass die daraus resultierenden Haarnadeln in ihrem doppelsträngigen Bereich die Erkennungssequenz für eine Restriktionsendonuklease aufweisen.

Die abschließende Aufreinigung der mittels des erfindungsgemäßen Verfahren hergestellten Vektoren erfolgt bevorzugt entweder durch Chromatographie oder Gelelektrophorese.

Wird der Promotor als Teil eines bakteriell amplifizierbaren Plasmides in das erfindungsgemäße Herstellungsverfahren eingesetzt, so handelt es sich bei der Restriktionsendonuklease, mit welcher der Promotor aus dem Plasmid ausgeschnitten werden kann, um ein Enzym der Gruppe der Klasse-II-Restriktionsendonukleasen, vorzugsweise aus der Gruppe BbsI, BbvI, BbvII, BpiI; BplI, Bsal, BsmAI, BsmBI, BsmFI, BspMI, Eam1104I, Earl, Eco31I, Esp3I, FokI, Hgal, SfaNI oder deren Isoschizomere.

Ein weiterer Gegenstand der vorliegenden Erfindung ist ein Kit zur Durchführung der erfindungsgemäßen Verfahren enthaltend wenigstens einen Promotor, haarnadelförmige Oligodesoxynukleotide und Enzyme. Bei den Enzymen handelt es sich um Ligasen, Restriktionsendonukleasen, Restriktionsexonukleasen, Kinasen und Polymerasen oder ausgewählte Kombinationen davon, in Form eines Enzymmixes. Zusätzlich kann der Kit je nach Ausführungsform noch Mittel zur Durchführung der enzymatischen Reaktionen enthalten sowie Mittel zur Aufreinigung der hergestellten Vektoren. Der Promotor kann im Kit als Teil eines Plasmids enthalten sein, aus dem dieser unter Verwendung einer geeigneten Restriktionsendonuklease herausgeschnitten werden kann.

Des Weiteren ist ein Kit vorgesehen, der die Synthese der multiplen RNAi-Expressionskonstrukte erlaubt, welche die beschriebene T-förmige Struktur enthalten. Dabei ist zu den bereits erwähnten Komponenten in dem Kit auch noch eine T-förmige Struktur enthalten, bei der in den doppelsträngigen Arm mit der zu transkribierenden Sequenz die gewünschte Sequenz inseriert werden kann.

Die erfindungsgemäßen Expressionskonstrukte zur gezielten Hemmung der Genexpression mittels RNA-Interferenz sind, sofern ein Linker mit Holliday-Junctions verwendet wird, zur Hemmung der Genexpression mehrer Gene geeignet. Aufgrund des speziellen Aufbaus der erfindungsgemäßen Konstrukte werden die zur Bildung der siRNA zu transkribierenden Sequenzen in gleichem Maß durch die RNA-Polymerase abgelesen.

Die Anzahl der Gene, die nach Transfektion eines erfindungsgemäßen multiplen Inhibitionskonstrukts gehemmt werden, hängt von der Anzahl der doppelsträngigen Arme ab, die der Linker aufweist.

Bezüglich der vorliegenden Erfindung ist weder die Kotransfektion von zwei separaten Vektoren noch die in der DE 100 48 417 A1 beschriebene Methode, die kodierenden Gensequenzen zusammen mit den notwendigen Steuerelementen (Promotor, poly(A)-Signal) chemisch miteinander zu verbinden, ein naheliegender Stand der Technik. Zwar werden bei der Kotransfektion mittels Lipofektion ebenfalls unterschiedliche Vektoren in räumliche Nähe zueinander gebracht und in die Zelle eingeschleust, jedoch ist das zugrundeliegende Prinzip dieser Methode ein anderes. Zudem entstehen nachteilhafterweise durch die beschriebene chemische Konjugation von Expressionskonstrukten Strukturen mit einem deutlich größeren Molekulargewicht, welche sich bekanntermaßen schwieriger in Zellen einschleusen lassen. Das gleiche trifft auch auf das in der WO 01/87348 A2 beschriebene Verfahren zum Aufbau eines Nukleinsäuren enthaltenden Dendrimers zu. Zusätzlich ist das dargestellte Verfahren zum Aufbau von Dendrimeren ein mehrstufiger und aufwendiger Herstellungsprozess.

Ein weiterer Gegenstand der vorliegenden Erfindung ist ein Arzneimittel, vorzugsweise eine Vakzine, welche eines der erfindungsgemäßen Expressionskonstrukte enthält. Derartige Arzneimittel können bei Mensch und Tier lokal und systemisch angewendet werden und sind zur Behandlung von Infektionen, beispielsweise Infektionen mit Herpesviren, Papillomaviren oder Lentiviren, zur Behandlung von Stoffwechselstörungen, wie beispielsweise der Parkinson'schen Krankheit oder der zystischen Fibrose, zur Behandlung von kardiovaskulären Erkrankungen, zur Behandlung von Krebserkrankungen, wie beispielsweise Karzinomen, Melanomen oder Sarkomen, zur Behandlung weiterer Krankheiten, wie beispielsweise der altersbedingten Makuladegeneration, und zur Behandlung nach Entzündungen, Verletzungen oder chirurgischen Eingriffen, die beispielsweise das Rückenmark, das Gehim oder andere Organe betreffen können, geeignet.

Weitere vorteilhafte Maßnahmen sind in den übrigen Unteransprüchen beschrieben; die Erfindung wird anhand von Ausführungsbeispielen und der nachfolgenden Figuren näher beschrieben; es zeigt:
- **Fig. 1**: Bestandteile der linearen siRNA-Vektoren
- **Fig. 2**: Bestandteile der T-förmigen siRNA-Vektoren
- **Fig. 3**: Bestandteile der T-förmigen Di-siRNA-Vektoren

- **Fig. 4**: Bestandteile der T-förmigen Triple-siRNA-Vektoren
- **Fig. 5**: Detaillierte Darstellung des Targetsequenzloops der T-förmigen Konstrukte
- **Fig. 6/7**: Experimentelle Ergebnisse des Vergleichs erfindungsgemäßer, T-förmiger siRNA Expressionsvektor (T-SiRNA-CDC2) mit synthetischer RNA (CDC2-RNA), die die identischen Targetsequenzen enthalten.

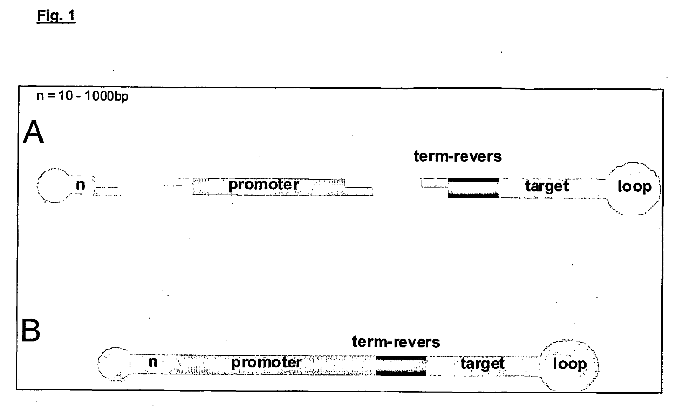

**Fig.1****:** zeigt die Bestandteile der linearen siRNA-Vektoren
**A:** In das Verfahren einzusetzende siRNA-Sequenz, die homolog zur Target-mRNA ist. Die Sequenz besteht aus einem sense oder antisense Bereich (target), einem 8 - 12 Basen langen Sequenzloop und einer revers komlpementären Terminationssequenz. Die siRNA-Sequenz kann aus einzelnen ODN-Fragmenten, die mit Hilfe des Enzymmix annealt, ligiert u. ggf. phosphoryliert werden müssen, bestehen, sie kann auch schon als vollständiges ODN-Fragment vorliegen.
   Desweiteren die Bestandteile, die durch das Enzym Ligase an das ODN-Fragment ligiert werden. Dabei handelt es sich um die Promotorsequenz mit entsprechenden komplementären Überhängen und um ein haarnadelförmiges Oligodinukleotid, dessen wiederum komplementär und einzigartiger Überhang von jeweils 4 Basen dazu führt, das ein kovalent geschlossener linearer Vektor entsteht, der aus Promotor, Targetsequenz und Terminationssequenz besteht und an den Enden haarnadelförmig geschlossen ist.
   Zwischen Promoter und abschließendem Oligonukleotid kann ein 10 bis 1000 Basen (= n) langer DNA-Doppelstrang einer nichtkodierenden Sequenz mit kurzen überstehenden Enden einzelsträngiger DNA eingesetzt werden.
**B:** zur Transfektion bereites Endprodukt

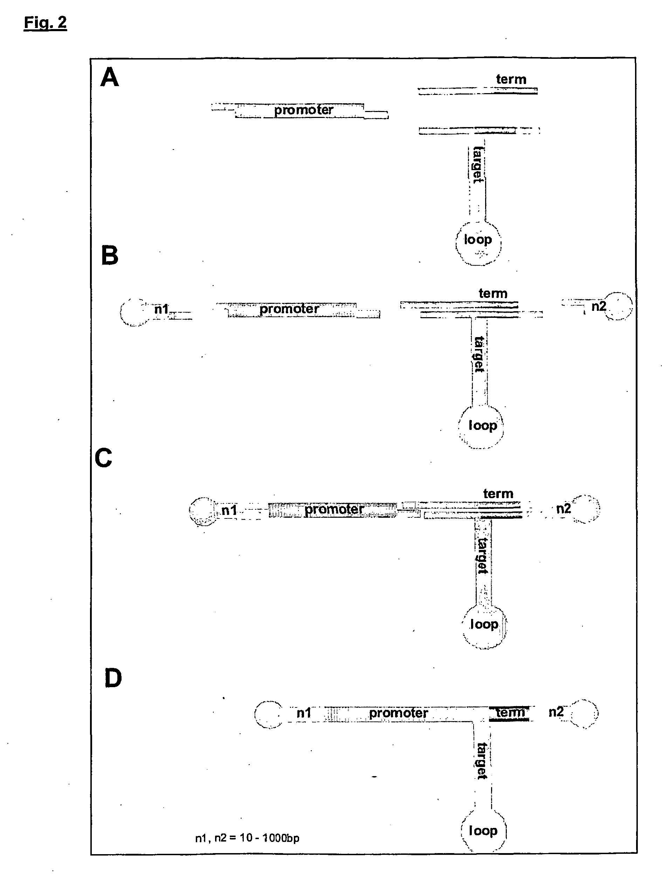

**Fig. 2****:** zeigt die Bestandteile der T-förmigen siRNA-Vektoren
**A:** In das Verfahren einzusetzende siRNA-Sequenz, die homolog zur Target-mRNA ist. Die Sequenz besteht aus dem sense oder antisense Bereich (target), einem 8 - 12 Basen langen Sequenzloop und einer Terminationssequenz. Die siRNA-Sequenz kann aus einzelnen ODN-Fragmenten, die mit Hilfe des Enzymmix annealt, ligiert u. ggf. phosphoryliert werden müssen, bestehen, sie kann auch schon als vollständiges ODN-Fragment vorliegen.
**B/C:** Desweiteren die Bestandteile, die durch das Enzym Ligase an das ODN-Fragment ligiert werden. Dabei handelt es sich um die Promotorsequenz mit entsprechenden komplementären Überhängen und um haarnadelförmige Oligodinukleotide, deren wiederum komplementären und einzigartigen Überhänge von jeweils 4 Basen dazu führt, das ein kovalent geschlossener Vektor entsteht, der aus Promotor, Senseloop oder Antisenseloop und Terminationssequenz besteht und an den Enden haarnadelförmig geschlossen ist.
   Zwischen Promoter und abschließendem Oligonukleotid, sowie zwischen Terminationssequenz und abschließendem Oligonukleotid kann ein 10 bis 1000 Basen (= n) langer DNA-Doppelstrang einer nichtkodierenden Sequenz mit kurzen überstehenden Enden einzelsträngiger DNA eingesetzt werden.
**D:** zur Transfektion bereites Endprodukt

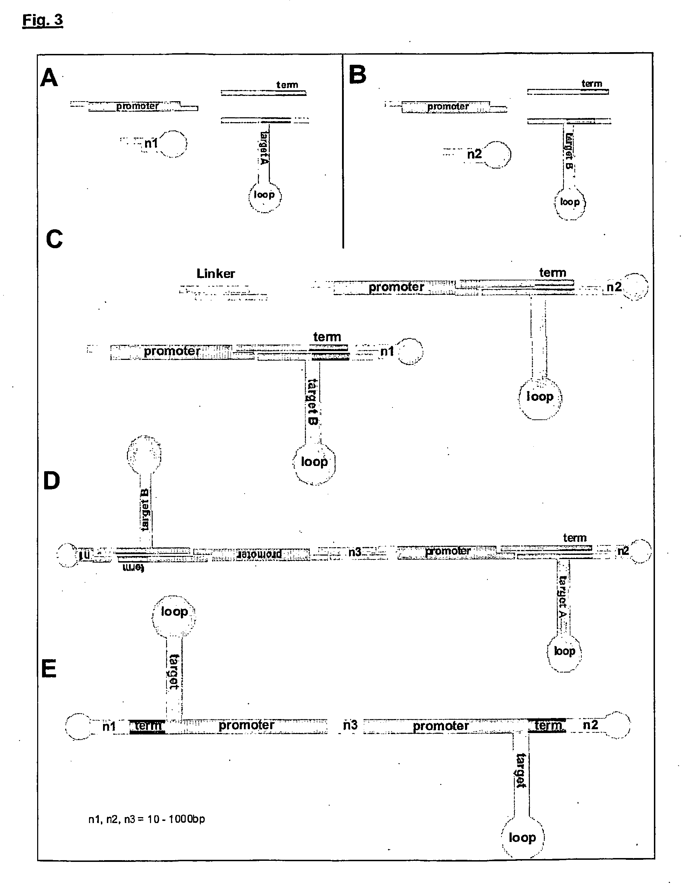

**Fig. 3****:** zeigt die Bestandteile der T-förmigen Multi-siRNA-Vektoren (Di-Konstrukt)
**A/B/C/D:** In das Verfahren einzusetzende siRNA-Sequenzen, die homolog zur Target-mRNA sind. Die zwei Sequenzen bestehen aus dem sense oder antisense Bereich (target A, target B), einem 8 - 12 Basen langen Sequenzloop und einer Terminationssequenz. Die siRNA-Sequenzen können aus einzelnen ODN-Fragmenten, die mit Hilfe des Enzymmix annealt, ligiert u. ggf. phosphoryliert werden müssen, bestehen, sie können auch schon als vollständiges ODN-Fragment vorliegen. Desweiteren die Bestandteile, die durch das Enzym Ligase an das ODN-Fragment ligiert werden. Dabei handelt es sich um die Promotorsequenz mit entsprechenden komplementären Überhängen, um einen DNA-Doppelstrang-Linker und um haarnadelförmige Oligodinukleotide, deren wiederum komplementären und einzigartigen Überhänge von jeweils 4 Basen dazu führt, das ein kovalent geschlossener Vektor entsteht, der aus Promotor, Senseloop oder Antisenseloop und Terminationssequenz besteht und an den Enden haarnadelförmig geschlossen ist.
   Zwischen Promoter und abschließendem Oligonukleotid, zwischen Terminationssequenz und abschließendem Oligonukleotid sowie jeweils zwischen Linker und Promoter kann ein 10 bis 1000 Basen (= n) langer DNA-Doppelstrang einer nichtkodierenden Sequenz mit kurzen überstehenden Enden einzelsträngiger DNA eingesetzt werden.
**E:** zur Transfektion bereites Endprodukt

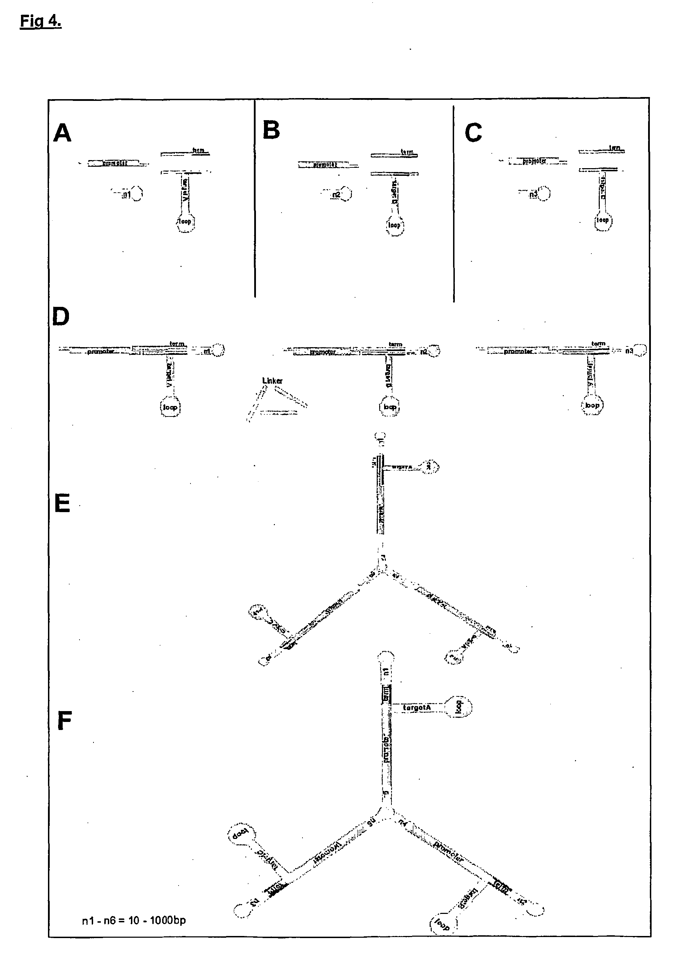

**Fig. 4****:** zeigt die Bestandteile der T-förmigen Multi-siRNA-Vektoren (Tri-Konstrukt)
**A/B/C/D/E:** In das Verfahren einzusetzende siRNA-Sequenzen, die homolog zur Target-mRNA sind. Die drei Sequenzen bestehen aus dem sense oder antisense Bereich (target A, target B), einem 8 - 12 Basen langen Sequenzloop und einer Terminationssequenz. Die siRNA-Sequenzen können aus einzelnen ODN-Fragmenten, die mit Hilfe des Enzymmix annealt, ligiert u. ggf. phosphoryliert werden müssen, bestehen, sie können auch schon als vollständiges ODN-Fragment vorliegen. Desweiteren die Bestandteile, die durch das Enzym Ligase an das ODN-Fragment ligiert werden. Dabei handelt es sich um die Promotorsequenz mit entsprechenden komplementären Überhängen, um einen DNA-Doppelstrang-Linker und um haarnadelförmige Oligodinukleotide, deren wiederum komplementären und einzigartigen Überhänge von jeweils 4 Basen dazu führt, das ein kovalent geschlossener Vektor entsteht, der aus Promotor, Senseloop oder Antisenseloop und Terminationssequenz besteht und an den Enden haarnadelförmig geschlossen ist.
   Zwischen Promoter und abschließendem Oligonukleotid, zwischen Terminationssequenz und abschließendem Oligonukleotid sowie jeweils zwischen Linker und Promoter kann ein 10 bis 1000 Basen (= n) langer DNA-Doppelstrang einer nichtkodierenden Sequenz mit kurzen überstehenden Enden einzelsträngiger DNA eingesetzt werden.
**F:** zur Transfektion bereites Endprodukt

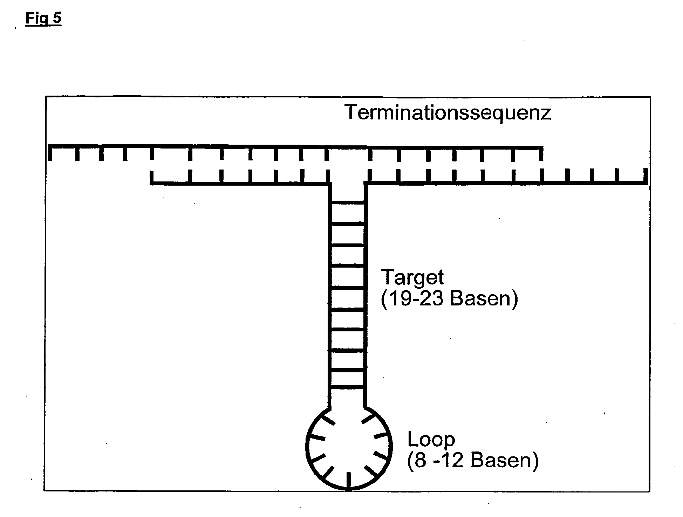

**Fig. 5** zeigt eine detaillierte Darstellung des Targetsequenzloops der T-förmigen Konstrukte.

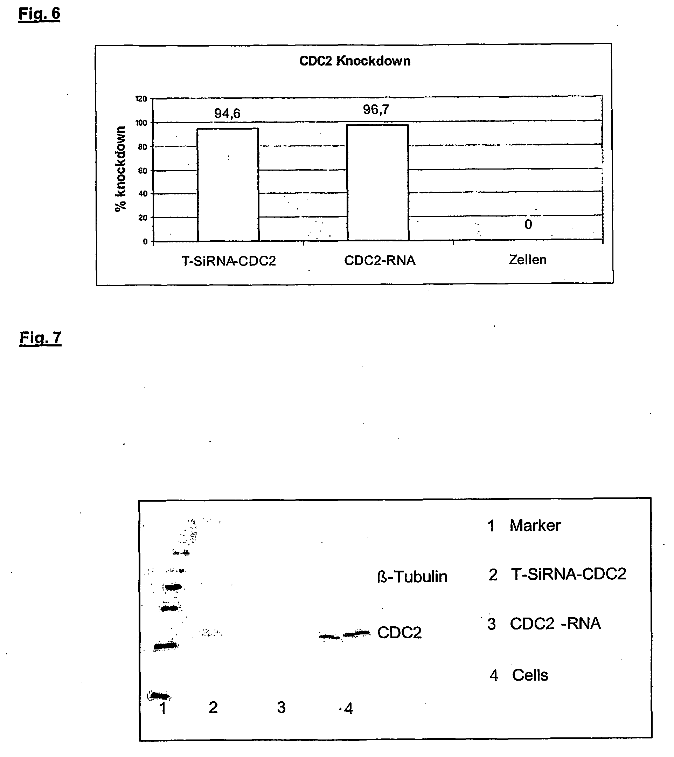

**Fig. 6****:** zeigt die Ergebnisse eines Experiments, bei dem der erfindungsgemäße T-förmige siRNA Expressionsvektor (T-SiRNA-CDC2) mit synthetischer RNA (CDC2-RNA) verglichen wird, die die identischen Targetsequenzen enthalten.
CDC2 mRNA wurde mittels quantitativer Real-time PCR nach Transfektion von HELA-Zellen bestimmt. Eingesetzt wurden: mit erfindungsgemäßem Verfahren hergestellter T-förmiger siRNA-Vektor, der eine CDC2 Targetsequenz beinhaltet, synthetische RNA derselben CDC2 Targetsequenz und unbehandelte Zellen. Die Werte stellen Mittelwerte dar, die aus Mehrfachbestimmungen errechnet wurden. Bei den unbehandelten Zellen wurde die Unterdrückung der CDC2 mRNA erwartungsgemäß nicht beobachtet. Dagegen zeigten die mit dem T-förmigen siRNA Vektor sowie der synthetischen RNA behandelten Zellen eine signifikant geringere CDC2 mRNA Menge. Diese ist bis zu 96,7% herabgesetzt im Vergleich zur Positivkontrolle (unbehandelte Zellen).

**Fig. 7** zeigt die Ergebnisse eines Experiments, bei dem der erfindungsgemäße T-förmige siRNA Expressionsvektor (T-SiRNA-CDC2) mit synthetischer RNA (CDC2-RNA) verglichen wird, die die identischen Targetsequenzen enthalten.
CDC2 Protein wurde mittels Western blot nach Transfektion von HELA-Zellen bestimmt. Eingesetzt wurden: mit erfindungsgemäßem Verfahren hergestellter T-förmiger siRNA-Vektor, der eine CDC2 Targetsequenz beinhaltet, synthetische RNA derselben CDC2 Targetsequenz und unbehandelte Zellen. Als interner Standard wurde β-Tubilin verwendet. Bei den unbehandelten Zellen (Bande 4) wurde die Unterdrückung der CDC2 mRNA erwartungsgemäß nicht beobachtet. Dagegen zeigten die mit dem T-förmigen siRNA Konstrukt (Bande 2) sowie der synthetischen RNA (Bande 3) behandelten Zellen eine signifikant geringere CDC2 Protein Menge.
Die Unterdrückung der Genexpression durch die T-förmigen SiRNA Konstrukte, welche mit einem "siRNA Expression Vector Kit" unter Verwendung des erfindungsgemäßen Verfahrens hergestellt wurden, ist vergleichbar mit den Effekten der synthetischen RNA-Transfektionen. Die Unterdrückung der Genexpression liegt bei beiden Transfektionen im Bereich zwischen 94 - 96%.

### Beispiel 1: Herstellung vom T-förmigen siRNA-Vektor zur Unterdrückung der CDC2 Expression

Der für die siRNA der CDC2 kodierende Vektor wurde wie folgt erhalten:

Die zwei ODN-Fragmente für CDC2 wurden bei 90°C für 3min erhitzt und durch langsames Abkühlen annealt. Dadurch wurde folgende für CDC2 kodierende Sequenz erhalten:
Seq. ID 1: TGGGGTCAGCTCGTTACTCTCTC

Dabei bilden die 19 Basen den sense Strang, die folgenden 4 Basen (unterstrichen) den Anfang der Loopsequenz.

Die Phosphorylierung durch PNKinase erfolgte im Anschluß. Zum Erhalt von 10 Mikrogramm Endprodukt wurden 3,9 Mikrogramm CDC2 eingesetzt. Nach Zugabe von 5,2 Mikrogramm H1-Promotor (Seq. ID 2) sowie der 5'-phosphorylierten haarnadelförmigen Oligodesoxynukleotide (Seq. ID 3 und 4): Seq. ID 3: 5' -PH-GGG AAA GCT TAG TTT TCT AAG CTT-3' (1,3 Mikrogramm) und
Seq. ID 4: 5` PH-GTT GGA ATT CAG TTT TCT GAA TTC-3' (1,3 Mikrogramm)
wurden mit Hilfe des Enzymes T4-DNA Ligase die einzelnen Fragmente ligiert. Das resultierende Gemisch an Nukleinsäuren wurde mit dem Enzym T7-DNA-Polymerase behandelt. Das Endprodukt, der siRNALuc exprimierende Vektor, wurde durch Säulenchromatographie aufgereinigt und stand zur Transfektion bereit.

## Patentansprüche

1. DNA-Expressionskonstrukt zur gezielten Hemmung der Genexpression mittels RNA-Interferenz bestehend aus folgende Komponenten
a) einer ersten DNA-Abschlusshaarnadelstruktur, bei der ein Einzelstrang aus Desoxyribonukleotiden derart zu einem doppelsträngigen Bereich rückgefaltet ist, dass dieser an einem Ende einen kohäsiven Überhang aufweist und am entgegengesetzten Ende einen einzelsträngigen Loop,
b) einer zweiten Abschlusshaarnadelstruktur, die analog zur ersten Abschlusshaarnadelstruktur aufgebaut ist,
c) wenigstens einer T-förmigen DNA-Struktur mit drei doppelsträngigen DNA-Armen, welche über Holliday-Junctions verbunden sind, wobei
i. ein Arm die zu transkribierenden Sequenzen enthält und die Einzelstränge dieses DNA-Doppelstrangarmes an dem Ende, welches der Verbindung mit den anderen Armen entgegengesetzt ist, durch einen einzelsträngigen DNA-Loop miteinander verbunden sind, und
ii. die beiden anderen Arme kohäsive Enden aufweisen, wobei ein Arm eine geeignete Terminationssequenz aufweist und der andere Arm eine geeignete Promotorsequenz.

2. DNA-Expressionskonstrukt zur gezielten Hemmung der Genexpression mittels RNA-Interferenz bestehend aus folgenden Komponenten
a) einer ersten DNA-Abschlusshaarnadelstruktur, bei der ein Einzeistrang aus Desoxyribonukleotiden derart zu einem doppelsträngigen Bereich rückgefaltet ist, dass dieser ein Ende mit kohäsivem Überhang aufweist und am entgegengesetzten Ende die den Doppelstrang bildenden Einzelstränge durch einen einzelsträngigen Loop miteinander verbunden sind,
b) einer zweiten Abschlusshaarnadelstruktur, die analog zur ersten Abschlusshaarnadelstruktur aufgebaut ist, und deren doppelsträngiger und einzelsträngiger Bereich aus der zu transkribierenden Sequenz besteht
c) einer geeigneten Promotorsequenz und einer geeigneten revers-komplementären Terminationssequenz, die sich zwischen den beiden unter a) und b) beschriebenen Abschlusshaarnadelstrukturen in einem DNA-Doppelstrang befinden.

3. DNA-Expressionskonstrukt nach Anspruch 1, wobei mehrere T-förmige DNA-Strukturen jeweils an einem Ende durch einen Linker mittels Holliday-Junctions verbunden sind.

4. DNA-Expressionskonstrukt nach Anspruch 2, wobei mehrere lineare DNA-Strukturen jeweils an einem Ende durch einen Linker mittels Holliday-Junctions verbunden sind,

5. DNA-Expressionskonstrukt nach wenigstens einem der vorhergehenden Ansprüche, wobei die kohäsiven Enden einzelner Komponenten zur gezielten Verbindung mit anderen Komponenten spezifisch und unterschiedlich gewählt sind.

6. DNA-Expressionskonstrukt zur gezielten Hemmung der Expression eines oder mehrerer Gene nach wenigstens einem der Ansprüche 1 bis 4, wobei die die zu transkribierenden Sequenzen für identische oder unterschiedliche Gene kodieren.

7. DNA-Expressionskonstrukt zur gezielten Hemmung der Expression eines oder mehrerer Gene nach wenigstens einem der Ansprüche 1 bis 4, wobei der Schutz vor Exonukleaseabbau durch einen kurzen Einzelstrangbereich aus drei bis acht Desoxynukleotiden erreicht wird, der die beiden Stränge des linearen Doppelstranges kovalent miteinander verknüpft.

8. DNA-Expressionskonstrukt zur gezielten Hemmung der Expression eines oder mehrerer Gene nach wenigstens einem der Ansprüche 1 bis 4, wobei dieses in dem kurzen Einzelstrangbereich mit einem oder mehreren Peptiden, Proteinen, Kohlenhydraten, Antikörpern oder Steroiden kovalent verknüpft ist.

9. Herstellungsverfahren für Vektoren, die nach ihrer Transfektion in eukaryote Zeilen die Bildung definierter Proteine gezielt durch RNA-Interferenz inhibieren, **gekennzeichnet durch** die folgenden Verfahrensschritte
a) Mischung eines DNA-Doppelstranges, welcher eine 19 - 23 Basen lange, singuläre Kopie einer Gensequenz oder ihre revers-kamplementäre Sequenz sowie ein Terminationssignal für RNA-Polymerasen enthält und **durch** einen 8-12 Basen langen Sequenzloop an einem Ende abgeschlossen ist, welcher so gewählt ist, dass gegenüberliegende Basen in keinem Fall komplementär zueinander sind und die flankierenden DNA-Einzelstränge zu einem DNA-Doppelstrang miteinander verbunden sind, der am anderen Ende einen kurzen überstehenden DNA Einzelstrang aufweist, mit
b) einem haarnadelförmigen Oligodesoxynukleotid, welches arm Ende kurze überstehende Enden einzelsträngiger DNA aufweist und
c) einem 10 bis 1000 Basen (= n) langen DNA-Doppelstrang einer nichtkodierenden Sequenz mit kurzen überstehenden Enden einzelsträngiger DNA, wobei das einzelsträngige 3'-Ende mit dem einzelsträngigen 5'-Ende des Promoters paaren kann und das einzelsträngige 5'-Ende komplementär zu dem einzelsträngigen 3'-Ende des haamadelförmigen Oligodesoxynukleotid ist, und
d) einem Promotor mit kurzen überstehenden Enden einzelsträngiger DNA, wobei das einzelsträngige 5'-Ende des Promotors mit dem haarnadelformigen Oligodesoxynukleotid oder dem 1.0 bis 1000 Basen-langem Doppelstrang paaren kann und das einzelsträngige 3'-Ende des Promotors komplementär zu dem einzelsträngigen 5'-Ende des unter a) beschriebenen DNA-Doppelstranges ist und
e) anschließender Ligation der DNA-Fragmente,
f) sowie abschließender Aufreinigung der hergestellten Vektoren.

10. Herstellungsverfahren für Vektoren, die nach ihrer Transfektion in eukaryote Zellen die Bildung definierter Proteine gezielt durch RNA-Interferenz inhibieren, **gekennzeichnet durch** die folgenden Verfahrensschritte
a) Mischung eines T-förmigen DNA-Doppelstranges, welcher eine 19 - 23 Basen lange, singuläre Kopie einer Gensequenz in 5' - 3'-Richtung sowie ein Terminationssignal für RNA-Polymerasen enthält und **durch** einen 8 - 12 Basen langen Sequenzloop an einem Ende abgeschlossen ist, welcher so gewählt ist, dass gegenüberliegende Basen in keinem Fall komplementär zueinander sind und die flankierenden Doppelstrangbereiche so **durch** zwei DNA-Einzelstränge miteinander verbunden sind und am anderen Ende zwei kurze überstehende DNA-Einzeistränge aufweist, und einem Oligodesoxynukleotid dessen Sequenz komplementär zu den beiden kurzen überstehenden DNA-Einzelsträngen ist und zwei kurze überstehenden Enden einzelsträngiger DNA bildet, mit
b) einem haarnadelförmigen Oligodesoxynukleotid, welches am Ende kurze überstchende Enden einzelsträngiger DNA aufweist und
c) einem weiteren haarnadelförmigen Oligodesoxynukleotid, welches am Ende kurze überstehende Enden einzelsträngiger DNA aufweist, komplementär zu dem T-förmigen DNA-Doppelstrang aus a) und
d) einem Promotor mit kurzen. überstehenden Enden einzelsträngiger DNA, wobei das einzelsträngige 5'-Ende des Promotors mit dem haarnadelförmigen Oligodesoxynukleotid aus b) paaren kann und das einzelsträngige 3'-Ende des Promotors komplementär zu dem einzelsträngigen 5'-Ende des T-förmigen. DNA-Doppelstranges ist und
e) anschließender Ligation der DNA-Fragmente, sowie
f) abschließender Aufreinigung der hergestellten Vektoren.

11. Herstellungsverfahren nach Anspruch 10, wobei nach Verfahrenschritt c) ein 10 bis 1000 Basen (= n) langer DNA-Doppelstrang einer nichtkodierenden Sequenz mit kurzen überstehenden Enden einzelsträngiger DNA zugegeben wird, wobei das einzelsträngige 3'-Ende mit dem einzelsträngigen 5'-Ende des Promoters paaren kann und das einzelsträngige 5'-Ende komplementär zu dem einzelsträngigen 3'-Ende des haarnadelförmigen Oligodesoxynukleotid aus Verfahrensschritt b) ist und/oder der Zugabe eines 10 bis 1000 Basen (= n) langen. DNA-Doppelstranges einer nichtkodierenden Sequenz mit kurzen überstehenden Enden einzelsträngiger DNA, wobei das einzelsträngige 5'-Ende mit dem einzelsträngigen 3'-Ende des T-förmigen DNA-Doppelstranges paaren kann und das einzelsträngige 3'-Ende komplementär zu dem einzelsträngigen 5'-Ende des haarnadelförmigen Oligodesoxynukleotid aus Verfahrensschritt c) ist.

12. Herstellungsverfahren nach wenigstens einem der Ansprüche 9 bis 11, wobei vor Beginn des Verfahrens partiell zueinander komplementäre einzelsträngige Oligodesoxynuklcotide zu einem Verbindungsmolekül (Linker) hybridisiert werden, wobei die Hybridisierung derart unvollständig erfolgt, dass zwei oder mehr doppelsträngige Bereiche mit kohäsiven Enden mit gleicher oder unterschiedlicher Basensequenz durch Holliday-Junctions miteinander verbunden werden und anschließender Durchführung der angegebenen Verfahrensschritte.

13. Verfahren nach weinigstens einem der Ansprüche 9 bis 12, wobei der Promotor Teil eines bakteriell amplifizierbaren Plasmides ist, welches vor der Mischung der jeweiligen Komponenten mit einer Reflüktionsendonuklease ausgeschnitten wird, welche eine den Promotor auf dem Plasmid flankierende Schnittstelle erkennt, die auf dem herzustellenden Expressionskonstrukt nicht vorhanden ist.

14. Verfahren nach Anspruch 13, wobei der jeweilige Ligationsschritt in Anwesenheit der Restriktionsendonuklease erfolgt, mit welcher der Promotor aus dem Plasmid gespalten wurde.

15. Verfahren nach wenigstens einem der Ansprüche 13 oder 14, wobei vor dem abschließenden Aufreinigungsschritt ein Verdau der Reaktionsmischung mittels einer ausschließlich für 3'-oder 5'-DNA-Enden spezifischen Exonuklease erfolgt.

16. Verfahren nach wenigstens einem der Ansprüche 13 bis 15, wobei es sich bei der Restriktionsendonuklease um ein Enzym der Gruppe der Klasse-II-Restriktionsendonukleasen, vorzugsweise ein Enzym aus der Gruppe BbsI, BbvI, BbvII, BpiI; Bp1I, BsaI, BsmAI, BsmBI, BsmFI, BspMI, Eam1104I, EarI, Echo31I, Esp3I, FokI, HgaI, SfaNI oder deren Isoschizomere handelt.

17. Verfahren nach wenigstens einem der Ansprüche 9 bis 16, wobei bei der Mischung der Komponenten ein DNA-Doppelstrang zugegeben wird, der aus dem partiellen Annealing von einem partiell selbstkomplementären Oligodesoxynukleotid oder wenigstens zwei Oligodesoxynukleotiden resultiert.

18. Verfahren nach wenigstens einem der Ansprüche 9 bis 17, wobei die haamadelformigen Oligodsoxynukleotide in ihrem doppelsträngigen Bereich die Erkennungssequenz für eine Restriktionsendonuklease aufweisen.

19. Verfahren nach wenigstens einem der Anspruche 9 bis 18, wobei die Aufreinigung durch Chromatographie und/oder mittels Gelelektrophorese erfolgt.

20. Kit zur Herstellung eines Expressionskonsbuktes gemäß einem der Ansprüche 1 bis 4, enthaltend zumindest zur Bildung von Abschlusshaarnadelstrukturen geeignete Oligodesoxynukleotide, zur Bildung eines Linkers geeignete Oligodesoxynukleotide, T-formige DNA-Strukturen in weiche die zu transkribierenden Sequenzen inseriert werden können sowie Enzyme und Mittel zur Durchführung von Ligation-, Spaltungs-, DNA-Degradations- und Aufreinigungsschritten

## Claims

1. DNA expression construct for the targeted inhibition of gene expression by means of RNA interference, consisting of the following components:
a) a first terminal DNA hairpin loop, in which one single strand of deoxyribonucleotides is folded back on itself to form a double-stranded region so that the latter exhibits a cohesive overhang at one end and a single-stranded loop at the opposite end,
b) a second terminal hairpin loop that is constructed analogously to the first terminal hairpin loop,
c) at least one T-shaped DNA structure with three double-stranded DNA arms that are linked via Holliday junctions to each other, wherein
i. one arm contains the sequences to be transcribed and the single strands of this DNA double-stranded arm are linked to one another at the end that is opposite to the junction with the other arms by a single-stranded DNA loop, and
ii. the two other arms exhibit cohesive ends, wherein one arm exhibits a suitable termination sequence and the other arm a suitable promoter sequence.

2. DNA expression construct for the targeted inhibition of gene expression by means of RNA interference, consisting of the following components:
a) a first terminal DNA hairpin loop, in which one single strand of deoxyribonucleotides is folded back on itself to form a double-stranded region so that the latter exhibits one end with a cohesive overhang and at the opposite end the single strands forming the double strand are linked to each other by a single-stranded loop,
b) a second terminal hairpin loop that is constructed analogously to the first terminal hairpin loop and whose double-stranded and single-stranded regions consist of the sequence to be transcribed,
c) a suitable promoter sequence and a suitable reverse complementary termination sequence, which are located in a DNA double strand between the two terminal hairpin loops described under a) and b).

3. DNA expression construct according to claim 1, wherein several T-shaped DNA structures are connected in each case at one end by a linker via Holliday junctions.

4. DNA expression construct according to claim 2, wherein several linear DNA structures are connected in each case at one end by a linker via Holliday junctions.

5. DNA expression construct according to at least one of the preceding claims, wherein the cohesive ends of individual components for the targeted linkage with other components are selected to be specific and distinct.

6. DNA expression construct for targeted inhibition of expression of one or several genes according to at least one of the claims 1 to 4, wherein the sequences to be transcribed code for identical or different genes.

7. DNA expression construct for targeted inhibition of expression of one or several genes according to at least one of the claims 1 to 4, wherein the protection against exonuclease degradation is achieved by means of a short single-stranded region of three to eight deoxynucleotides, which covalently links the two strands of the linear double-strand to each other.

8. DNA expression construct for targeted inhibition of expression of one or several genes according to at least one of the claims 1 to 4, wherein the latter is covalently linked in the short single-stranded region to one or several peptides, proteins, carbohydrates, antibodies or steroids.

9. Method for the synthesis of vectors that after their transfection into eukaryotic cells inhibit in a targeted manner the formation of defined proteins by means of RNA interference, **characterized by** the following method steps:
a) mixing of a DNA double strand that contains a 19 to 23 base long, singular copy of a gene sequence or its reverse complementary sequence and a termination signal for RNA polymerases, and is closed at one end by an 8 to 12 base long sequence loop, which is selected such that opposite bases are never complementary to each other and the flanking DNA single strands are linked to each other to form a DNA double strand, which exhibits at its other end a short protruding DNA single strand, with
b) a hairpin-shaped oligodeoxynucleotide, which exhibits at its end short protruding ends of single-stranded DNA, and
c) a 10 to 1000 base (= n) long DNA double strand of a non-coding sequence with short protruding ends of single-stranded DNA, wherein the single-stranded 3'-end can pair with the single-stranded 5'-end of the promoter and the single-stranded 5'-end is complementary to the single-stranded 3'-end of the hairpin-shaped oligodeoxynucleotide, and
d) a promoter with short protruding ends of single-stranded DNA, wherein the single-stranded 5'-end of the promoter can pair with the hairpin-shaped oligodeoxynucleotide or with the 10 to 1000 base long double strand, and the single-stranded 3'-end of the promoter is complementary to the single-stranded 5'-end of the DNA double strand described under a), and
e) following ligation of the DNA fragments,
f) and final purification of the synthesized vectors.

10. Method for the synthesis of vectors that after their transfection into eukaryotic cells inhibit in a targeted manner the formation of defined proteins by means of RNA interference, **characterized by** the following method steps:
a) mixing of a T-shaped DNA double strand that contains a 19 to 23 base long, singular copy of a gene sequence in the 5' to 3' direction and a termination signal for RNA polymerases, and which is closed at one end by an 8 to 12 base long sequence loop, which is selected such that opposite bases are never complementary to one another and the flanking double-stranded regions are linked with each other by two DNA single strands, and at the other end exhibits two short protruding DNA single strands, and an oligodeoxynucleotide whose sequence is complementary to the two short protruding DNA single strands and forms two short protruding ends of single-stranded DNA, with
b) a hairpin-shaped oligodeoxynucleotide, which exhibits at its end short protruding ends of single-stranded DNA, and
c) a further hairpin-shaped oligodeoxynucleotide, which exhibits at its end short protruding ends of single-stranded DNA complementary to the T-shaped DNA double strand from a), and
d) a promoter with short protruding ends of single-stranded DNA, wherein the single-stranded 5'-end of the promoter can pair with the hairpin-shaped oligodeoxynucleotide from b) and the single-stranded 3'-end of the promoter is complementary to the single-stranded 5'-end of the T-shaped DNA double strand, and
e) following ligation of the DNA fragments, and
f) final purification of the synthesized vectors.

11. Method for synthesis according to claim 10, wherein after method step c) a 10 to 1000 base (= n) long DNA double strand of a non-coding sequence with short protruding ends of single-stranded DNA is added, wherein the single-stranded 3'-end can pair with the single-stranded 5'-end of the promoter and the single-stranded 5'-end is complementary to the single-stranded 3'-end of the hairpin-shaped oligodeoxynucleotide from method step b) and/or the addition of a 10 to 1000 base (= n) long DNA double strand of a non-coding sequence with short protruding ends of single-stranded DNA, wherein the single-stranded 5'-end can pair with the single-stranded 3'-end of the T-shaped DNA double strand and the single-stranded 3'-end is complementary to the single-stranded 5'-end of the hairpin-shaped oligodeoxynucleotide from method step c).

12. Method for synthesis according to at least one of the claims 9 to 11, wherein before the beginning of the method partially complementary single-stranded oligodeoxynucleotides are hybridized to form a linker molecule, wherein the hybridization is incomplete such that two or more double-stranded regions with cohesive ends with the same or different base sequence are linked to each other by Holliday junctions and following this the method steps indicated are carried out.

13. Method according to at least one of the claims 9 to 12, wherein the promoter is part of a bacterially amplifiable plasmid that before the mixing of the respective components is excised using a restriction endonuclease that recognises a restriction site flanking the promoter on the plasmid, a restriction site which does not exist on the expression construct to be synthesized.

14. Method according to claim 13, wherein the respective ligation step occurs in the presence of the restriction endonuclease with which the promoter was spliced from the plasmid.

15. Method according to at least one of the claims 13 or 14, wherein before the final purification step a digestion of the reaction mixture using an exonuclease exclusively specific for 3' or 5' DNA ends takes place.

16. Method according to at least one of the claims 13 to 15, wherein the restriction endonuclease is an enzyme of the group of class II restriction endonucleases, preferably an enzyme from the group BbsI, BbvI, BbvII, BpiI; BplI, BsaI, BsmAI, BsmBI, BsmFI, BspMT, Eam1104I, EarI, Eeo31I, Esp3I, FokI, HgaI, SfaNI or their isoschizomers.

17. Method according to at least one of the claims 9 to 16, wherein a DNA double strand is added to the mixture of components, a strand which results from the partial annealing of a partially self-complementary oligodeoxynucleotide or at least two oligodeoxynucleotides.

18. Method according to at least one of the claims 9 to 17, wherein the hairpin-shaped oligodeoxynucleotides exhibit the recognition sequence for a restriction endonuclease in their double-stranded region.

19. Method according to at least one of the claims 9 to 18, wherein purification is carried out by chromatography and/or using gel electrophoresis.

20. Kit for the synthesis of an expression construct pursuant to one of the claims 1 to 4, containing at least oligodeoxynucleotides suitable for the formation of terminal hairpin loops, oligodeoxynucleotides suitable for the formation of a linker, T-shaped DNA structures in which the sequences to be transcribed can be inserted, and enzymes and means of carrying out steps of ligation, splicing, DNA degradation and purification.

## Revendications

1. Construction d'expression d'ADN pour l'inhibition spécifique de l'expression de gène par interférence ARN, comprenant les composants suivants :
a) une première structure ADN terminale en épingle à cheveux, dans laquelle un simple brin de désoxyribonucléotides est replié sur lui-même pour former une région à double brin, présentant à une extrémité un recouvrement cohésif et une boucle à simple brin à l'extrémité opposée,
b) une deuxième terminale structure en épingle à cheveux, construite de manière analogue à la première structure terminale en épingle à cheveux,
c) au moins une structure ADN en forme de T, avec trois bras d'ADN à double brin, qui sont reliés les uns aux autres par des jonctions Holliday, dans lequel
i. un bras comprend les séquences qui doivent être transcrites, et les simples brins de ce bras d'ADN à double brin sont reliés l'un l'autre, au niveau de l'extrémité qui est opposée à la jonction avec les autres bras, par une boucle ADN à simple brin, et
ii. les deux autres bras ont des extrémités cohésives, un bras ayant une séquence de terminaison adaptée et l'autre bras ayant une séquence de promoteur adaptée.

2. Construction d'expression d'ADN pour l'inhibition spécifique de l'expression de gène au moyen d'interférence ARN, comprenant les composants suivants :
a) une première structure ADN terminale en épingle à cheveux, dans laquelle un simple brin de désoxyribonucléotides est replié sur lui-même, pour former une région à double brin, de sorte que la région à double brin présente une extrémité avec un recouvrement cohésif et, à l'extrémité opposée, les simples brins formant le double brin sont reliés l'un à l'autre par une boucle à brin unique,
b) une deuxième structure terminale en épingle à cheveux, construite de manière analogue à la première boucle terminale en épingle à cheveux, et dont les régions à simple brin et à double brin comprennent la séquence qui doit être transcrite,
c) une séquence de promoteur adaptée et une séquence de terminaison complémentaire inversée, situées dans un double brin d'ADN entre les deux structures terminales en épingle à cheveux décrites au point a) et au point b).

3. Construction d'expression d'ADN selon la revendication 1, dans lequel plusieurs structures ADN en forme de T sont reliées respectivement au niveau d'une extrémité, par un lieur, par l'intermédiaire de jonctions Holliday.

4. Construction d'expression d'ADN selon la revendication 2, dans lequel plusieurs structures ADN linéaires sont reliées respectivement au niveau d'une extrémité, par un lieur, par l'intermédiaire de jonctions Holliday.

5. Construction d'expression d'ADN selon l'une quelconque des revendications précédentes, dans lequel les extrémités cohésives des composants individuels pour la liaison ciblée avec d'autres composants sont sélectionnées de manière à être spécifiques et distinctes.

6. Construction d'expression d'ADN pour l'inhibition spécifique d'un ou plusieurs gènes selon au moins l'une des revendications 1 à 4, dans lequel les séquences qui doivent être transcrites sont codantes pour des gènes identiques ou différents.

7. Construction d'expression d'ADN pour l'inhibition spécifique de l'expression d'un ou plusieurs gènes selon au moins l'une des revendications 1 à 4, dans lequel la protection contre la dégradation exonucléase est réalisée au moyen d'une région courte à simple brin de 3 à 8 désoxynucléotides, qui relient l'un à l'autre de manière covalente les deux brins du double brin linéaire.

8. Construction d'expression d'ADN pour l'inhibition spécifique de l'expression d'un ou plusieurs gènes selon au moins l'une des revendications 1 à 4, dans lequel celui-ci est relié de manière covalente dans la région courte à simple brin à un ou plusieurs peptides, protéines, carbohydrates, anticorps ou stéroïdes.

9. Procédé de fabrication de vecteurs qui, après leur transfection dans des cellules eucaryotes, empêche, par interférence ARN, de manière ciblée, la formation de protéine définie, **caractérisé par** les étapes de procédé suivantes :
a) mélange d'un double brin d'ADN qui contient une copie unique, comprenant 19 à 23 bases, d'une séquence de gène ou sa séquence complémentaire inversée, et un signal de terminaison pour polymérases ARN, et qui est fermé à une extrémité par une boucle de séquence longue de 8 à 12 bases, qui est choisie de sorte que les bases opposées ne sont en aucun cas complémentaires l'une l'autre, et les simples brins d'ADN qui l'encadrent sont reliés les uns aux autres pour former un double brin d'ADN, comprenant au niveau de son autre extrémité un simple brin d'ADN court faisant saillie, avec
b) un oligodésoxynucléotide en forme de boucle en épingle à cheveux, comprenant au niveau d'une extrémité des extrémités courtes faisant saillie de simple brin d'ADN, et
c) un double brin d'ADN de 10 à 1000 bases (= n) de long, d'une séquence non codante, avec des extrémités courtes faisant saillie d'ADN à simple brin dans lequel l'extrémité 3' à simple brin peut former une paire avec l'extrémité 5' à simple brin du promoteur, et l'extrémité 5' à simple brin est complémentaire de l'extrémité 3' à simple brin de l'oligodésoxynuctéotide en forme de boucle en épingle à cheveux, et
d) un promoteur avec des extrémités courtes faisant saillie d'ADN à simple brin, dans lequel l'extrémité 5' à simple brin du promoteur peut former une paire avec l'oligodésoxynucléotide en forme de boucle en épingle à cheveux ou avec le double brin ayant une longueur entre 10 à 1000 bases, et l'extrémité 3' à simple brin du promoteur est complémentaire de l'extrémité 5' à simple brin du double brin d'ADN décrit au point a), et
e) ligature suivante des fragments d'ADN,
f) et purification finale des vecteurs synthétisés.

10. Procédé de fabrication de vecteurs qui, après leur transfection dans des cellules eucaryotes, empêche de manière spécifique la formation de protéines définies, au moyen d'interférence ARN, **caractérisé par** les étapes de procédé suivantes :
a) mélange d'un double brin d'ADN en forme de T, contenant une copie singulière, comprenant 19 à 23 bases, d'une séquence de gène dans la direction 5' vers 3', et un signal de terminaison pour polymérases ARN, et qui est fermé au niveau d'une extrémité par une boucle de séquence de 8 à 12 bases, qui est choisie de sorte que des bases opposées ne sont en aucun cas complémentaires l'une l'autre, et les régions à double brin qui l'encadrent sont reliées l'une l'autre par deux simples brins d'ADN, et, au niveau de l'autre extrémité, qui comprend deux simples brins d'ADN faisant saillie, et un oligodésoxynucléotide dont la séquence est complémentaire aux deux simples brins d'ADN courts faisant saillie, et qui forment deux extrémités faisant saillie courtes d'ADN à simple brin, avec
b) un oligodésoxynucléotide en forme de boucle en épingle à cheveux, qui présente à son extrémité des extrémités faisant saillie courtes d'ADN à simple brin, et
c) un oligodésoxynucléotide additionnel en forme de boucle en épingle à cheveux, qui présente à son extrémité des extrémités courtes faisant saillie d'ADN à simple brin complémentaire au double brin d'ADN en forme de T du point a), et
d) un promoteur avec des extrémités faisant saillie courtes d'ADN à simple brin, dans lequel l'extrémité 5' à simple brin du promoteur peut former une paire avec l'oligodésoxynueléotide en forme de boucle en épingle à cheveux du point b), et l'extrémité 3' à simple brin du promoteur est complémentaire de l'extrémité 5' à simple brin du double brin d'ADN en forme de T et,
e) ligature suivante des fragments d'ADN, et
f) purification finale des vecteurs synthétisés.

11. Procédé de fabrication selon la revendication 10, dans lequel, après l'étape de procédé c), un double brin d'ADN comprenant 10 à 1000 bases (= n) d'une séquence non codante avec des extrémités en saillie courtes d'ADN à simple brin est ajouté, dans lequel l'extrémité 3' à simple brin peut former une paire avec l'extrémité 5' à simple brin du promoteur, et l'extrémité 5' à simple brin est complémentaire de l'extrémité 3' à simple brin de l'oligodésoxynuctéotide en forme de boucle en épingle à cheveux de l'étape de procédé b) et/ou l'addition d'un double brin d'ADN comprenant 10 à 1000 bases (= n) d'une séquence non codante, avec des extrémités courtes faisant saillie d'ADN à simple brin, dans lequel l'extrémité 5' à simple brin peut former une paire avec l'extrémité 3' à simple brin du double brin d'ADN en forme de T, et l'extrémité 3' à simple brin est complémentaire de l'extrémité 5' à simple brin de l'oligodésoxynucléotide en forme de boucle en épingle à cheveux de l'étape de procédé c).

12. Procédé de fabrication selon au moins l'une des revendications 9 à 11, dans lequel, avant le début du procédé, des oligodésoxynucléotides à simple brin partiellement complémentaires sont hybridés pour former une molécule de liaison (lieur), dans lequel l'hybridation est incomplète de sorte que deux ou plusieurs régions à double brin avec des extrémités cohésives, avec la même séquence de base ou une séquence de base différente, deviennent reliées l'une l'autre par des jonctions Holliday, et à la suite de cela, les étapes du procédé indiqué sont réalisées.

13. Procédé selon au moins l'une des revendications 9 à 12, dans lequel le promoteur est en partie un plasmide amplifiable de bactéries qui, avant le mélange des composants respectifs, est coupé en utilisant une endonucléase de restriction qui reconnait un site de coupe encadrant le promoteur sur le plasmide, le site de coupe n'existant pas sur le produit de recombinaison qui doit être synthétisé.

14. Procédé selon la revendication 13, dans lequel l'étape de ligature respective a lieu en présence de l'endonucléase de restriction avec laquelle le promoteur a été séparé du plasmide.

15. Procédé selon au moins l'une des revendications 13 ou 14, dans lequel, avant l'étape de purification finale, une digestion du mélange de réaction a lieu en utilisant une exonucléase spécifique exclusivement pour les extrémités d'ADN 3' ou 5'.

16. Procédé selon au moins l'une des revendications 13 à 15, dans lequel l'endonucléase de restriction est une enzyme du groupe des endonucléases de restriction de classe II, de préférence une enzyme du groupe Bbsl, Bbvl, BbvII, BpiII BplI, BsaI, BsmAI, BsmBI, BsmFI, BspMI, Fam1104I, Earl, Eco31I, Esp3I, FokI, Hgal, SfaNI ou de leurs Isoschisomères.

17. Procédé selon au moins l'une des revendications 9 à 16, dans lequel un double brin d'ADN est ajouté au mélange des composants, le double brin résultant de l'annulation partielle d'un oligodésoxynucléotide partiellement autocomplémentaire ou d'au moins deux oligodésoxynucléotides.

18. Procédé selon au moins l'une des revendications 9 ou 17, dans lequel les oligodésoxy-nuoléotides en forme de boucle en épingle à cheveux présentent la séquence de reconnaissance d'une endonucléase de restriction dans leur région à double brin.

19. Procédé selon au moins l'une des revendications 9 ou 18, dans lequel la purification est réalisée par chromatographie et/ou en utilisant électrophorèse de gel.

20. Kit pour la fabrication de construction d'expression selon l'une des revendications 1 à 4, contenant au moins des oligodésoxynucléotides adaptés pour la formation de structures terminales en épingle à cheveux, des oligodésoxynucléotides adaptés pour la formation d'un lieur, des structures ADN en forme de T dans lesquelles les séquences qui doivent être transcrites sont insérées, et des enzymes et moyens de réaliser des étapes de ligature, d'épissage, de dégradation d'ADN, et de purification.


### SEQUENCE LISTING

<110> Mologen AG
<120> DNA-Konstrukte zur spezifischen Hemmung der Genexpression durch RNA-Interferenz
<130> XI 1165-06
<150> EP 05025727.8
   <151> 2005-11-25
<160> 4
<170> PatentIn version 3.3
<210> 1
   <211> 23
   <212> DNA
   <213> Artificial
<220>
   <223> synthetisches Oligodesoxynukleotid
<220>
   <221> misc_feature
   <223> first 19 bases are sense CDC2, last 4 bases are beginning of loop
<400> 1
   tggggtcagc tcgttactct ctc
   23
<210> 2
   <211> 99
   <212> DNA
   <213> Homo sapiens
<220>
   <221> misc_feature
   <223> Promotor of Human Gene for H1 RNA
<400> 2
<210> 3
   <211> 24
   <212> DNA
   <213> Artificial
<220>
   <223> synthetisches Oligodesoxynukleotid
<220>
   <221> misc_feature
   <223> 5-prime phosphorylated loop-oligodeoxynecleotide
<400> 3
   gggaaagctt agttttctaa gctt
   24
<210> 4
   <211> 24
   <212> DNA
   <213> Artificial
<220>
   <223> synthetsiches Oligodesoxynukleotid
<220>
   <221> misc_feature
   <223> 5-prime phosphorylated loop-oligodeoxynucleotide
<400> 4
   gttggaattc agttttctga attc
   24